Europäisches Patentamt

European Patent Office

Office européen des brevets

(11) Publication number: **0 148 811
B1**

(12) **EUROPEAN PATENT SPECIFICATION**

(45) Date of publication of patent specification: **06.09.89**

(21) Application number: **85850007.7**

(22) Date of filing: **08.01.85**

(60) Divisional application 88850123 filed on 12.04.88.

(51) Int. Cl.⁴: **A 61 K 9/00,** A 61 K 47/00, A 61 K 9/14, A 61 K 9/16

(54) Pharmaceutical composition.

(30) Priority: **10.01.84 SE 8400085**

(43) Date of publication of application:
**17.07.85 Bulletin 85/29**

(45) Publication of the grant of the patent:
**06.09.89 Bulletin 89/36**

(84) Designated Contracting States:
**AT BE CH DE FR GB IT LI LU NL SE**

(56) References cited:
**EP-A-0 013 263
EP-A-0 040 590
EP-A-0 063 014
EP-A-0 080 341
EP-A-0 094 117
WO-A-83/00435
DE-A-2 510 620
DE-A-3 046 559**

**The file contains technical information
submitted after the application was filed and
not included in this specification**

(73) Proprietor: **LEJUS MEDICAL AKTIEBOLAG
Taljegardsgatan 3
S-431 33 Mölndal (SE)**

(72) Inventor: **Appelgren, Curt Henry
Benjaminssons väg P1 1461
S-434 00 Kungsbacka (SE)**
Inventor: **Eskilsson, Eva Christina
Körsbärsvägen 15
S-435 00 Mölnlycke (SE)**

(74) Representative: **Inger, Lars Ulf Bosson
L + U INGER Patentbyra HB Garvaregatan 12
S-262 00 Ängelholm (SE)**

Courier Press, Leamington Spa, England.

## Description

### Technical field

The present invention relates to a new oral, pharmaceutical composition having an improved release of the therapeutically active compound present.

The object of the present invention is to obtain a possibility to release therapeutically active compounds, and particularly to release such active compounds which show different solubilities in the pH range 1 to 8, and thereby relatively high solubility.

### Background of the invention

There exists an everlasting problem within pharmacy to be able to administer a therapeutically active compound as close as possible to the colon, or preferably in the colon in order thereby to eliminate the risk for acidic influence on the active compound by the gastric juice, or to prevent from irritation of the ventricular mucous membranes, or to obtain a therapeutically effect in the lower part of the gastrointestinal tract. A further problem is to obtain a steady, preferably linear release of a therapeutically active compound in order to give a steady blood plasma level of the therapeutically active compound, excluding initial release peaks, which may cause side-effects due to too a high concentration in the body of the therapeutically active compound.

In EP—A—0 040 590 it has been proposed an oral pharmaceutical composition comprising a core containing a therapeutically active compound, which core has been coated with a layer comprising 10 to 85% by weight of an anionic polymer soluble at a pH above 5.5 and 15 to 90% by weight of a water-insoluble polymer selected from the group of quaternary ammonium substituted acrylic polymers. Such a product gives, however, too high a permeability in gastric juice and is only suitable for therapeutically active compounds having a relatively low solubility in acidic environment. Thereby it is meant a solubility of <1 g/100 ml.

DE—A1—3,046,559 (=US—A—4,432,966, GB—A—2,066,070) relates to a compressed tablet for disintegration in the colon comprising an active ingredient-containing core coated with a first layer containing microcrystalline cellulose and a second layer containing an enteric organic polymer coating. The inner layer may thereby consist of microcrystalline cellulose and ethyl cellulose, an alkylphthalate and a solvent, and the outer, second layer may consist of cellulse acetylphthalate, and an alkylphthalate, whereby the cellulose acetyl phthalate can be exchanged with hydroxypropylmethylcellulose phthalate, benzophenyl salicylate, cellulose acetosuccinate, copolymers of styrene and of maleic acid, formylated gelatin, salol, keratin, stearic acid, myristic acid, gluten, acrylic and methacrylic resins, and copolymers of maleic acid and phthalic acid derivatives. The microcrystalline cellulose present in the inner first layer forms ducts through which the release of the active ingredient of the core can take place.

There is no real method or composition known today within pharmacy, which composition can save compounds being very soluble in acidic environment and provide them with an adequate release profile in the more neutral pH of the small intestine and the large intestine after an attack by gastric acid at the passage of the ventricle.

### Description of the present invention

It has now surprisingly been shown possible to be able to solve said problem by means of the present invention, which is characterized by a core comprising a therapeutically active substance in the form of a weak base or a weak acid and having a solubility of above 1 g/100 ml at pH 1 to 8, on which core there is provided a first, inner layer of a diffusion membrane in the form of ethyl cellulose and/or a copolymer of ethyl acrylate, methyl methacrylate, and trimethylammonium ethyl methacrylate chloride, and thereabove there is provided a second layer of at least one anionic polymer selected from hydroxypropylmethyl cellulose phthalate, cellulose acetate phthalate, polyvinyl acetate phthalate and copolymers of methacrylic acid and methacrylic acid methylester (Eudragit® L100; acid:ester 1:1) having a $pk_a$ of 4.5 to 7, preferably 5.5 to 6.5.

Further characteristics are evident from the accompanying claims.

By means of the present invention the core is protected against attack by gastric juice after the administration by means of the outer layer comprising an anionic polymer having a $pk_a$ of 4.5 to 7, whereafter, when this outer layer has been removed by dissolution at the passage of the small intestine having its higher pH, a diffusion takes place so that a slow but controlled release takes place of the therapeutically active compound through the diffusion membrane due to the difference in concentrations on each side of said membrane. The release takes thereby place at such a rate that 80—90% of the therapeutically active compound has been released after 7 to 10 h, which means that the release can take place in a constant, pH-independent way, and thereby in a very reproducible way.

The present invention applies to therapeutically active compounds which are weak bases or weak acids, and which, in the pH range of 1 to 8, have a varying solubility exceeding 1 g/100 ml at maximal solubility in said range. As will be evident from the following a diffusion membrane can not prevent a rapid release in e.g. acidic environment, when coating an active compound which has a high solubility in acidic environment, but 40 to 50% of the active compound is released within 2 h.

Such therapeutically active compounds which have such a varying solubility within pH 1 to 8 and a solubility, which maximally exceeds 1 g/100 ml in said range are quinidine sulphate, quinidine bisulphate, quinidine gluconate, quinidine hydrochloride, metoprolol tartrate, metoprolol fumarate, furosemide, propranolol, alprenolol, 5 - aminosalicylic acid, and others. I.e. such weak bases and weak acids or salts of these, the $pk_a$ of which is 1 to 8.

The core comprising therapeutically active compounds is a granule having a diameter of 0.1 to 2.5 mm, which granule can consist of a therapeutically active compound in the form of a crystalline product, or compacted product only, or can consist of a therapeutically active compound in combination with pharmaceutically indifferent compounds such as lactose, mannitol, sugar, microcrystalline cellulose, starch, and waxes.

The diffusion membrane consists, as mentioned above, of ethyl cellulose and/or a copolymer of ethyl acrylate (63—65%), methyl methacrylate (31.7—32.3%), and trimethylammonium ethyl methacrylate chloride (2.5—5%), which copolymer is sold under the name Eudragit with quality identifications RL and RS, whereby RL denotes a product having 5% of said methacrylate chloride, an RS denotes a product having 2.5% of said methacrylate chloride. Ethyl cellulose is suitably mixed with Eudragit RL and RS in the relation 20:80 to 80:20. The diffusion membrane can also contain a permeability improving compound, particularly in these cases when the therapeutically active compound is difficult to dissolve at a higher pH, i.e. at such a pH, which is present in the lower part of the gastrointestinal duct including the small intestine. Such permeability improving compounds are hydroxypropyl cellulose (Klucel®), methyl cellulose (Methocel®), hydroxypropyl methyl cellulose (Pharmacoat®), polyethylene glycol, polyvinyl pyrrolidone (PVP), and fatty acids, and others, respectively.

The outer layer consists as mentioned, of one or more anionic polymers having a $pk_a$ of 4.5 to 7. These anionic polymers are cellulose acetate phthalate, polyvinyl acetate phthalate, hydroxypropyl methyl cellulose phthalate, methyl methacrylate methylester (such as Eudragit L100).

The membranes/layers are normally applied in an amount of at least 5 g per $m^2$ of particle area divided between the two layers, suitably 5 to 80 $g/m^2$ particle area, using 2.5 to 60 $g/m^2$ per layer, and at least 2.5 $g/m^2$.

From the point of view of flavour and/or identification a flavoured or coloured layer can optionally be applied outside the two release controlling layers. This is, however, no part of the present invention as such.

When dosing the ready made product a number of discrete, coated particles/granules corresponding to a therapeutical dosage unit of the actual therapeutical compound are administered.

When administering, in order to achieve a steady blood plasma level of the therapeutically active compound, a split dosage unit of the therapeutically active compound provided with a coating according to the present invention is administered together with some particles/granules which are not coated, and some particles/granules coated with a diffusion membrane only, whereby the release takes place in the gastric juice, and/or a retarded granulate having a release time of 1 to 2 h can be used, whereby such granules/particles are coated with an anionic polymer having a $pk_a$ of 4.5 to 7. Thus particles/granules having a laminate type coating according to the present invention can be mixed with particles/granules without any coating and with particles/granules having a diffusion membrane only or an anionic polymer coating only in different relationships depending on the actual desired blood level of therapeutically active compound.

The particles/granules are normally packed in small envelopes, tubular containers, or other capsules comprising a dose unit of a therapeutically active compound.

## Example 1

Granulated quinidine sulphate having a granular size of 0.6 to 1.5 mm and comprising 60% of quinidine sulphate were sprayed with a solution of 50% by weight of ethyl cellulose, and 50% by weight of ethyl acrylate-methyl methacrylate - trimethylammonium ethyl methacrylate chloride copolymer with the weight-% 65:32.5:2.5, (Eudragit RL) dissolved in methylene chloride/isopropanol in a fluidized bed. The concentration of polymer in the solvent mixture was 4% by weight.

The granules obtained provided with a diffusion membrane were then provided with a layer of hydroxypropyl cellulose phthalate (HP-55) dissolved in methylene chloride/ethanol-mixture by means of the corresponding technique.

The inner diffusion membrane was applied in an amount of 12 $g/m^2$ and the outer layer of anionic polymer was applied in an amount of 16 $g/m^2$.

## Example 2

Granules were coated in accordance with Example 1 above with layers of the same materials and material mixtures.

The inner diffusion layer was thereby applied in an amount of 11.3 $g/m^2$, and the outer layer of anionic polymer was applied in an amount of 14.3 $g/m^2$.

## Example 3

In a first test the release rate for a composition according to Examples 1 and 2 above were compared with a composition provided with a diffusion membrane only, whereby the diffusion membrane had the

3

same composition as the diffusion membranes of the compositions of Examples 1 and 2 above, and was applied in an amount of 11.6 g/m$^2$. The quinidine granulate was from the same batch in all three Examples.

The release rates were determined in gastric juice of pH 1 only; after pre-exposure in gastric juice of pH 1 for 2 h, and then a phosphate buffer of pH 6.5, and in phosphate buffer of pH 6.5, only, respectively. The values obtained are given in Table 1 below.

TABLE 1

| Composition | Gastric juice pH 1 | | | | | Gastric juice+phosphate buffer pH 1        pH 6.5 | | | | | | Phosphate buffer pH 6.5 | | | | | | |
|---|---|---|---|---|---|---|---|---|---|---|---|---|---|---|---|---|---|---|
| | 1 | 2 | 3 | 4 | 5 | 1 | 2 | 3 | 4 | 5 | 9 | 1 | 2 | 3 | 4 | 5 | 6 | 9h |
| Ex. 1 | 2 | 2 | 4 | 4 | 5 | 1 | 1 | 8 | 18 | 29 | 65 | 14 | 26 | 38 | 45 | 53 | 60 | 75 |
| Ex. 2 | 5 | 7 | 11 | 13 | 15 | 6 | 7 | 16 | 24 | 35 | 77 | 11 | 24 | 35 | 47 | 56 | 64 | 84 |
| Ex. 3 | 24 | 47 | 66 | 78 | 88 | 20 | 40 | 46 | 48 | 49 | 52 | 6 | 9 | 12 | 15 | 17 | 20 | 30 |

The figures give the percent of quinidine sulphate released.

As evident from the above given results a pure diffusion membrane can not prevent a release in gastric juice depending on the fact that the solubility of the quinidine sulphate is very high in acidic environment and that a concentration difference will occur between the solution which is formed inside the membrane by the penetrating gastric juice and the surrounding solution, the gastric juice. Further, a diffusion membrane only gives a very slow release in pH 6.5-buffer, whereby only about 30% was released in case of only pH 6.5-buffer, and 52% was released in case of pre-exposure in pH 1, 2 h, followed by an accompanying exposure in pH 6.5-buffer. This means that a decreased biological availability of the compound is obtained, which is very unsatisfactory. However, there will be obtained a very adequate release in case an outer layer of anionic polymer has been applied. To obtain a protection against a release in gastric juice is *per se* reasonable, as this polymer has a pk$_a$ between 4.5 and 7, i.e. it is undissolvable in an acidic pH, but is dissolved and eliminated in a neutral pH. However, it is very surprising that quite another release profile is obtained at pH 6.5 in this case compared with the case of a diffusion membrane only. The differences there, but can not be explained today.

## Claims

1. Oral pharmaceutical composition having an improved release of the therapeutically active compound present independent from its solubility, characterized by a core comprising a therapeutically active compound in the form of a weak base or a weak acid having a solubility of at least 1 g/100 ml at pH 1 to 8, on which core there is applied a first inner layer of a diffusion membrane consisting of ethyl cellulose and/or a copolymer of ethyl methacrylate - methyl methacrylate - trimethylammonium ethyl methacrylate chloride, and thereabove there is applied a second layer of at least one anionic polymer selected from hydroxypropylmethyl cellulose, phthalate, cellulose acetate phthalate, polyvinyl acetate phthalate, and copolymers of methacrylic acid and methacrylic acid methylester having a pk$_a$ of 4.5 to 7.

2. Pharmaceutically composition according to claim 1, characterized in that the pk$_a$ of the material in the second layer is 5.5 to 6.5.

3. Pharmaceutical composition according to claim 1, characterized in that the diffusion membrane further comprises a permeability improving compound selected from hydroxypropyl cellulose, hydroxypropyl methyl cellulose, methyl cellulose, polyethylene glycol, polyvinyl pyrrolidone and fatty acids.

4. Pharmaceutical composition according to claim 1, characterized in that the diffusion membrane is a mixture of ethyl cellulose, and a copolymer of ethyl methacrylate - methyl methacrylate - trimethyl ammonium ethylmethacrylate chloride in the weight relationship 80:20 to 20:80, whereby the weight relationship between the monomers of the copolymer is 63 to 65:31.7 to 32.3:2.5 to 5.

5. Pharmaceutical composition according to claims 1—4, characterized in that the active compound is quinidine sulphate, quinidine bisulphate, quinidine gluconate, quinidine hydrochloride, metoprolol tartrate, metoprolol fumarate, furosemide, 5-amino salicylic acid, propranolol, alprenolol, or pharmaceutically acceptable salts thereof and/or other weak bases, weak acids, or salts thereof.

6. Pharmaceutical composition according to claims 1—5, characterized in that the core comprising the therapeutically active compound is a particle/granule having a diameter of 0.1 to 2.5 mm; that the first inner diffusion membrane is applied in an amount of at least 2.5 g per m$^2$ of particle area, and that the second layer is applied in an amount of at least 2.5 g per m$^2$ of particle area.

7. Process for the preparation of a pharmaceutical composition having an improved release of a therapeutically active compound, characterized in that a core comprising a therapeutically active compound in the form of a weak base or a weak acid having a solublity of at least 1 g/100/ml at pH 1 to 8, is coated with an inner layer of a diffusion membrane comprising ethyl cellulose, and/or a copolymer of ethyl methacrylate - methyl methacrylate - trimethylammonium ethyl methacrylate chloride by applying a solution containing said compound(s), and is coated with a second layer of at least one anionic polymer selected from hydroxypropylmethyl cellulose phthalate, cellulose acetate phthalate, polyvinyl acetate phthalate, and copolymers of methacrylic acid and methacrylic acid methylesters having a pk$_a$ of 4.5 to 7.

## Patentansprüche

1. Orale pharmazeutische Zusammensetzung mit einer verbesserten Freisetzung der vorhandenen therapeutisch wirksamen Verbindung unabhängig von ihrer Löslichkeit, gekennzeichnet durch einen Kern, der eine therapeutisch wirksame Verbindung in der Form einer schwachen Base oder einer schwachen Säure mit einer Löslichkeit von wenigstens 1 g/100 ml bei pH 1 bis 8 umfaßt, wobei auf diesem Kern eine erste innere Schicht einer Diffusionsmembran, die aus Ethylcellulose und/oder einem Copolymer von Ethylmethacrylat - Methylmethacrylat - Trimethylammoniummethylmethacrylatchlorid besteht, aufgebracht ist und darüber eine zweite Schicht wenigstens eines anionischen Polymers aufgebracht ist, das unter Hydroxypropylmethylcellulosephthalat, Celluloseacetatphthalat, Polyvinylacetatphthalat und Copolymeren von Methacrylsäure und Methacrylsäuremethylester mit einem pk$_a$ von 4,5 bis 7 ausgewählt ist.

2. Pharmazeutische Zusammensetzung nach Anspruch 1, dadurch gekennzeichnet, daß der pk$_a$ des Materials in der zweiten Schicht 5,5 bis 6,5 ist.

3. Pharmazeutische Zusammensetzung nach Anspruch 1, dadurch gekennzeichnet, daß die Diffusionsmembran außerdem eine permeabilitätsverbessernde Verbindung umfaßt, die unter Hydroxypropylcellulose, Hydroxypropylmethylcellulose, Methylcellulose, Polyethylenglycol, Polyvinylpyrrolidon und Fettsäuren ausgewählt ist.

4. Pharmazeutische Zusammensetzung nach Anspruch 1, dadurch gekennzeichnet, daß die Diffusionsmembran ein Gemisch von Ethylcellulose und eines Copolymers von Ethylmethacrylat - Methylmethacrylat - Trimethylammoniumethylmethacrylatchlorid im Gewichtsverhältnis 80:20 bis 20:80 ist, wobei das Gewichtsverhältnis zwischen den Monomeren des Copolymers 63 bis 65:31,7 bis 32,3:2,5 bis 5 ist.

5. Pharmazeutische Zusammensetzung nach Anspruch 1 bis 4, dadurch gekennzeichnet, daß die wirksame Verbinding Chinidinsulfat, Chinidinbisulfat, Chinidinglyconat, Chinidinhydrochlorid, Metoprololtartrat, Metoprololfumarat, Furosemid, 5 - Aminosalicylsäure, Propranolol, Alprenolol oder ein pharmazeutisch verträgliches Salz hiervon und/oder eine andere schwache Base, schwache Säure oder ein Salz hiervon ist.

6. Pharmazeutische Zusammensetzung nach Anspruch 1 bis 5, dadurch gekennzeichnet, daß der die therapeutisch wirksame Verbindung umfassende Kern ein Teilchen/Korn mit einem Durchmesser von 0,1 bis 2,5 mm ist, daß die erste innere Diffusionsmembran in einer Menge von wenigstens 2,5 g/m$^2$ Teilchenoberfläche aufgebracht ist und daß die zweite Schicht in einer Menge von wenigstens 2,5 g/m$^2$ Teilchenoberflächen aufgebracht ist.

7. Verfahren zur Herstellung einer pharmazeutischen Zusammensetzung mit verbesserter Freisetzung einer therapeutisch wirksamen Verbindung, dadurch gekennzeichnet, daß ein Kern, der eine therapeutisch aktive Verbindung in der Form einer schwachen Base oder einer schwachen Säure mit einer Löslichkeit von wenigstens 1 g/100 ml bei pH 1 bis 8 umfaßt, mit einer inneren Schicht einer Diffusionsmembran, die Ethylcellulose und/oder ein Copolymer von Ethylmethacrylat - Methylmethacrylat - Trimethylammoniumethylmethacrylatchlorid umfaßt, durch Aufbringung einer diese Verbindung(en) enthaltenden Lösung beschichtet wird und mit einer zweiten Schicht wenigstens eines anionischen Polymers überzogen wird, das unter Hydroxypropylmethylcellulosephthalat, Celluloseacetatphthalat, Polyvinylacetatphthalat und Copolymeren von Methacrylsäure und Methacrylsäuremethylestern mit einem pk$_a$ von 4,5 bis 7 ausgewählt ist.

## Revendications

1. Composition pharmaceutique utilisable par voie orale, présentant une libération améliorée du composé actif en thérapeutique présent indépendamment de sa solubilité, caractérisée par un noyau comprenant un composé actif en thérapeutique sous forme d'une base faible ou d'un acide faible ayant une solubilité d'au moins 1 g/100 ml au pH de 1 à 4, noyau sur lequel est appliquée une première couche interne d'une membrane de diffusion consistant en éthyl cellulose et/ou en un copolymère de méthacrylate d'éthyle, de méthacrylate de méthyle et de chlorure de méthacrylate d'éthyle de triméthyl ammonium, avec ensuite par-dessus une seconde couche d'au moins un polymère anionique choisi parmi le phtalate d'hydroxypropyl méthyl cellulose, l'acétate-phtalate de cellulose, l'acétate-phtalate de polyvinyle, et les copolymères d'acide méthacrylique et d'ester méthylique d'acide méthacrylique, ayant une valeur pk$_a$ de 4,5 à 7.

2. Composition pharmaceutique suivant la revendication 1, caractérisée en ce que la valeur pk$_a$ de la matière de la seconde couche est de 5,5 à 6,5.

3. Composition pharmaceutique suivant la revendication 1, caractérisée en ce que la membrane de diffusion comprend en outre un composé améliorant la perméabilité, choisi parmi l'hydroxypropyl cellulose, l'hydroxypropyl méthyl cellulose, la méthyl cellulose, le polyéthylène glycol, la polyvinyl pyrrolidone et les acides gras.

4. Composition pharmaceutique suivant la revendication 1, caractérisée en ce que la membrane de diffusion est un mélange d'éthyl cellulose et d'un compolymère de méthacrylate d'éthyle, de méthacrylate de méthyle, et de chlorure de méthacrylate d'éthyle de triméthyl ammonium dans le rapport en poids de 80/20 à 20/80, la relation de poids entre les monomères du copolymère étant de 63 à 65/31,7 à 32,3/2,5 à 5.

5. Composition pharmaceutique suivant l'une quelconque des revendications 1 à 4, caractérisée en ce que le composé actif est du sulfate de quinidine, du bisulphate de quinidine, du gluconate de quinidine, du chlorhydrate de quinidine, du tartrate de métoprolol, du fumarate de métoprolol, du furosémide, de l'acide 5 - aminosalicylique, du propranolol, de l'alprénolol ou des sels acceptables en pharmacie des composés précédentes, et/ou d'autres bases faibles, acides faibles ou leurs sels.

6. Composition pharmaceutique suivant l'une quelconque des revendications 1 à 5, caractérisée en ce que le noyau comprenant le composé actif en thérapeutique est une particule/granulé ayant un diamètre de 0,1 à 2,5 mm, en ce que la première membrane de diffusion interne est appliquée en une quantité d'au moins 2,5 g/m$^2$ d'aire de particule, et en ce que la seconde couche est appliquée en une quantité d'au moins 2,5 g/m$^2$ d'aire de particule.

7. Procédé de préparation d'une composition pharmaceutique présentant une libération améliorée d'un composé actif en thérapeutique, caractérisé en ce qu'un noyau comprenant un composé actif en thérapeutique, sous forme d'une base faible ou d'un acide faible ayant une solubilité d'au moins 1 g/100 ml au pH de 1 à 8, est enrobé par une couche interne d'une membrane de diffusion comprenant de l'éthyl cellulose, et/ou un copolymère de méthacrylate d'éthyle, de méthacrylate de méthyle et de chlorure de méthacrylate d'éthyle de triméthylammonium, par application d'une solution contenant ce ou ces composés, avec ensuite enrobage par une seconde couche d'au moins un composé anionique choisi parmi le phtalate d'hydroxypropyl méthyl cellulose, l'acétate-phtalate de cellulose, l'acétate-phtalate de polyvinyle, et des copolymères d'acide méthacrylique et d'esters méthyliques d'acide méthacrylique, ayant une valeur $pk_a$ de 4,5 à 7.